# EUROPÄISCHE PATENTANMELDUNG

(11) **EP 0 857 476 A1**
(43) Veröffentlichungstag der Anmeldung: **12.08.1998**
(21) Anmeldenummer: 97121524.9
(22) Anmeldetag: 06.12.1997
(51) Int. Cl.: A61K 7/06, A61K 7/13

(54) **Verwendung von Gerbstoffen zur Verlängerung der Haltbarkeit von Haartönungen**

(30) Priorität: 06.02.1997 DE 19704384
(71) Anmelder: Wella Aktiengesellschaft, 64274 Darmstadt (DE)
(72) Erfinder: Kischka, Karl-Heinz, Dr., 64293 Darmstadt (DE); Schröder, Friedel, 64293 Darmstadt (DE)

(57) **Zusammenfassung**

Verwendung von natürlichen oder synthetischen, organischen oder anorganischen Gerbstoffen in Haarbehandlungsmitteln zur Verlängerung der Haltbarkeit von Haartönungen.

## Beschreibung

Gegenstand der Erfindung ist die Verwendung von Gerbstoffen in Haarbehandlungsmitteln zur Verlängerung der Haltbarkeit von Haartönungen.

Aus der deutschen Offenlegungsschrift 32 33 665 ist bereits ein Mittel zur Haarbehandlung bekannt, das eine Polyphenolverbindung, ein Chelatisierungsmittel und Zersetzungsprodukte von Keratinen enthält und zur Verhinderung der Eluierung von Proteinen aus dem Haar bestimmt ist. Zu den dort genannten Polyphenolverbindungen gehören auch Tannine.

Weiterhin ist aus der US-Patentschrift 4 981 485 bekannt, daß durch die gleichzeitige Anwendung von Tanninen und wasserlöslichen Salzen von Zink, Kupfer, Zinn, Magnesium und Aluminium unterschiedliche und dauerhafte Haarfärbungen erzeugt werden können.

Keiner dieser Veröffentlichungen ist zu entnehmen, daß mit Hilfe eines Gerbstoffes die Dauerhaftigkeit von Haartönungen erhöht werden kann.

Es ist außerdem eine reichhaltige Auswahl von direkt ziehenden Farbstoffen bekannt, die je nach Molekülstruktur, Beschaffenheit der Haarstruktur und der Trägerbasis mehr oder weniger stark auf das Haar aufziehen und zusammen mit der bereits vorhandenen Grundfarbe eine neue Haartönung erzeugen. Hierfür werden Farbstoffe aus den Klassen der Nitro-, Azo-, Chinon- und Triphenylmethanfarbstoffe, außerdem auch Pflanzenfarbstoffe wie Henna und Reng eingesetzt.

Alle direkt ziehenden Farbstoffe mit Ausnahme der Nitrofarbstoffe dringen nur oberflächlich in das Haar ein. Sie lagern sich primär in der Schuppenschicht des Haares an. Der Cortex des Haares wird bestenfalls an-, aber nicht durchgefärbt. Diese Eigenschaft schmälert die Haltbarkeit der mit direkt ziehenden Farbstoffen erzeugten Haartönungen. Sie werden durch die notwendigen Haarwäschen abgeschwächt und nach einiger Zeit ganz ausgewaschen. Umwelteinflüsse wirken ähnlich.

Es stellte sich deshalb die Aufgabe, ein Mittel zur Verfügung zu stellen, das die Haltbarkeit von Haartönungen verlängert. Erfindungsgemäß wird diese Aufgabe durch die Verwendung von natürlichen oder synthetischen, organischen oder anorganischen Gerbstoffen in Haarbehandlungsmitteln erreicht.

Überraschenderweise können zur Verlängerung der Haltbarkeit von Haartönungen ganz unterschiedliche Gerbstoffe mit guten Erfolg eingesetzt werden. Gerbstoffe pflanzlichen Ursprunges, die Tannine, sind hierfür besonders gut geeignet. Sowohl die kondensierten Gerbstoffe, die sich vom Catechin ableiten, als auch die hydrolisierbaren Gerbstoffe oder Gallotannine, die sich von der Gallussäure ableiten, können erfindungsgemäß eingesetzt werden. Die Vertreter beider Gruppen von Gerbstoffen enthalten zahlreiche phenolische Hydroxygruppen und können aus Pflanzenmaterialien wie Eiche, Roßkastanie, Hamamelis, Kamille, Huflattich, Arnika, Katechu, Quebracho oder Mimosa, aber auch aus Pflanzengallen, Rhabarber und vielen anderen Pflanzen gewonnen werden. In Wasser lösen sich die Gerbstoffe natürlichen Urspungs kolloidal oder semikolloidal.

Auch synthetische organische Gerbstoffe, die als Syntane bezeichnet werden, eignen sich für die erfindungsgemäße Verlängerung der Haltbarkeit von Haartönungen. Bei den Syntanen handelt es sich um in der Regel durch Sulfonierung löslich gemachte Aldehyd-Kondensationsprodukte aromatischer Grundkörper, insbesondere von Phenol, Kresol, Naphthalin und Naphthol. Hierzu gehören auch Harnstoff- oder Melamin-Formaldehyd-Kondensationsprodukte.

Eine dritte Gruppe von erfindungsgemäß einsetzbaren Farbstoffen umfaßt wasserlösliche Chrom(III)-, Aluminium-, Zirkonium- oder Eisensalze, die in Form ihrer Chloride, Acetate, Formiate, Sulfate oder Phosphate Anwendung finden können. Auch Polyphosphate wie Pyrophosphate, Tripolyphosphate, Methaphosphate und Hexametaphosphate eignen sich zur Verlängerung der Haltbarkeit von Haartönungen.

Die vorstehend genannten Gerbstoffe werden einem Haarbehandlungsmittel in eine Menge von 0,01 bis 5, vorzugsweise in einer Menge von 0,1 bis 2 Gewichtsprozent zugesetzt.

Durch die Verwendung von Gerbstoffen in Haarbehandlungsmitteln kann die Haltbarkeit von Haartönungen verlängert werden, die insbesondere durch die folgenden, physiologisch unbedenklichen, direkt ziehenden Farbstoffe erzeugt werden:

### Nitrofarbstoffe (blau)

4-N-Ethyl-N-(2'-hydroxyethyl)amino-1-(2''-hydroxyethyl)amino-2-nitro-benzol, 1-Amino-3-methyl-4-(2'-hydroxyethyl)amino-6-nitro-benzol, 1-(2'-Hydroxyethyl)amino-2-nitro-4-bis-(2''-hydroxyethyl)amino-benzol, 4-Bis-(2'-hydroxyethyl)amino-1-(2''-methoxyethyl)amino-nitrobenzol, 1-(2',3'-Dihydroxypropyl) amino-2-nitro-4-[ethyl-(2''-hydroxyethyl)aminol-benzol, 1-[(2',3'-Dihydroxypropyl)amino]-2-nitro-4-[ethyl-2''-(hydroxyethyl)amino]-benzol, 1-(3'-Hydroxypropylamino)-2-nitro-4-bis-(2''-hydroxyethylamino)-benzol.

### Nitrofarbstoffe (rot)

1-Amino-4-(2'-hydroxyethyl)-amino-nitrobenzol, 1-Hydroxy-2-amino-4,6-dinitro-benzol, 1-Amino-2-nitro-4-bis-(2'-hydroxyethyl)amino-benzol,1-Amino-2-nitro-4-(2'-hydroxyethyl)amino-5-chlorbenzol, 1-(2'Hydroxyethyl)amino-2-nitro-4-amino-benzol, 1-Hydroxy-3-nitro-4-aminobenzol, 1-(2'-Aminoethyl)amino-2-nitro-4-(2''-hydroxyethyl)-oxybenzol,3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'-dihydroxypropyl)amino-5-chlor-2-nitro-benzol, 1-Hydroxy-2-(2'hydroxyethyl)amino-4,6-dinitro-benzol, 2-Amino-6-chlor-4-nitrophenol, 1-Hydroxy-3-nitro-4-(3'-hydroxypropylamino)-benzol, 3-Nitro-4-ethylamino-benzoesäure, 4-Amino-2-nitro-diphenylamino-2-carbonsäure,2-Chlor-6-ethylamino-4-nitrophe-nol, 2,5-Diamino-6-nitropyridin, 1,2,3,4-Tetrahydro-6-nitrochinoxalin.

### Nitrofarbstoffe (gelb)

4-(2'-Hydroxyethyl)amino-3-nitro-benzonitril, 4-(2'-Hydroxyethyl)amino-3-nitro-benzamid,1-Amino-2-(2'-hydroxyethyl)amino-5-nitro-benzol, 1-Methoxy-2-(2'-hydroxyethyl)amino-5-nitro-benzol, 1-Hydroxy-3-nitro-4-(2'-hydroxyethyl)amino-benzol, 1-Hydroxy-2-amino-3-nitro-benzol, 1-Amino-2-methyl-6-nitro-benzol, 1-(2'-Hydroxyethyl)-oxy-3-methylamino-4-nitrobenzoi, 1-Methylamino-2-nitro-5-(2',3'-dihydroxypropyl)-oxybenzol,1-(2'-Hydroxyethyl)amino-2-hydroxy-4-nitro-benzol,1-Methoxy-3-(2'-aminoethyl)-amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitrotrifluormethyl-benzol,2,4-Bis-[N-(2'-hydroxyethyl)amino]-5-chlor-nitrobenzol, 4-(2',3'-Dihydroxypropyl)amino-3-nitro-trifluormethyl-benzol, 4-(2'-Hydroxymethyl)amino-3-nitro-methylbenzol, 4-(2'-Hydroxyethyl)amino-3-nitro-chlorbenzol,

### Azofarbstoffe

1-(4'-Nitrophenylazo)-2-methyl-4-bis-(2''-hydroxyethyl)amino-benzol, 1-(3'-Nitro-4-amino)-phenylazo-2-hydroxy-7-trimethyl-ammoniumchlorid-naphthalin,1-(2'-Hydroxy-4'-sulfo-6'-nitro)-naphthylazo-2-hydroxynaphthalin,1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid,1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-l'-naphthyl)azo]-1-naphthalinsulfonsäure, 1-(4'-Sulfophenylazo)-2-hydroxy-naphthalin, 1-(4'-Sulfophenylazo)-2-hydroxy-6-sulfo-naphthalin, 4-Amino-[4'-bis-(2''-hydroxyethyl)amino-azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-l-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Di-methylphenylazo)-6-(4''-sulfophenylazo)-1,3-dihydroxy-benzol.

### Chinonfarbstoffe

1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'-methyl-phenyl )-amino-4-hydroxy-anthrachinon, 1,4-Diamino-anthrachinon, 1-Amino-2-sulfo-4-cyclohexylamino-anthrachinon, 1-Methylamino-4-aminopropyl-amino-anthrachinon, 1-Aminopropylamino-anthrachinon.

### Triphenylmethanfarbstoffe

4',4'',4'''-Triamino-3-methyl-triphenylcarboniumchlorid, Bis-(4,4-Diethylaminophenyl)-4'-ethylanlino-naphthyl-carboniumchlorid, Bis-(4,4-Dimethylaminophen)-4'-phenyl-amino-naphthyl-carboniumchlorid und 4,4-Bis-(N-Ethyl-3-sulfobenzyl)-amino-2''-sulfofuchsonium.

Auch die Haltbarkeit von natürlichen Farbstoffen wie Henna oder Reng wird erfindungsgemäß verbessert.

Die Anwendung des Gerbstoffes kann in Form unterschiedlicher Haarbehandlungsmittel erfolgen. Beispielsweise kann der Gerbstoff einer wäßrigen, wäßrig-alkoholischen oder alkoholischen Lösung, einer Emulsion, einem Shampoo, einem Gel oder einem Aerosol zugesetzt werden. Als Aerosol kann das gerbstoffhaltige Gemisch mit einem Treibgas oder mittels einer mechanisch betriebenen Sprühvorrichtung versprüht oder aufgeschäumt werden.

In dem gerbstoffhaltigen Haarbehandlungsmittel können weiterhin alle für derartige Zubereitungen bekannten und üblichen Zusatzstoffe wie Parfümöle, Komplexbildner, Wachse, Konservierungsstoffe, kosmetische Harze wie Polyvinylpyrrolidon oder Polyvinylacetat, Verdicker, Algenate, Guagum, haarpflegende Substanzen wie kationische Polymere oder Lanolinderivate, Netzmittel und Emulgatoren enthalten sein. Der pH-Wert einer derartigen Zubereitung liegt in dem für Haarkosmetika üblichen pH-Wert-Bereich von etwa 2 bis 9.

Die nachfolgenden Beispiele sollen den Gegenstand der Erfindung erläutern. Sie zeigen insbesondere, daß eine Verlängerung der Haltbarkeit von Haartönungen durch die Verwendung von Gerbstoffen unabhängig davon erreicht wird, ob diese vor, während oder nach der Tönungsbehandlung dem Haar zugeführt werden.

### Beispiel 1: Vorbehandlung des Haares mit Tanninlösung

Blondes Humanhaar, das bereits durch vorausgegange Haarbehandlungen oxidativ leicht geschädigt war, wurde mit 0,5%-iger wäßriger Tanninlösung (pH = 3,2) behandelt; anschließend wurde das Haar mit Tönungsmittel 1 weinrot gefärbt.

| **Tönungsmittel 1** (weinrote Tönung) | **Gew.-%** |
|---|---|
| Fettalkohol | 1,50 |
| Cetyltrimethylammoniumchlorid | 1,00 |
| Ethanol | 8,00 |
| 1-(2'-Hydroxyethyl)amin-2-nitro-4-bis-(2''-hydroxyethyl)aminobenzol | 1,00 |
| 1-Amino-5-chlor-4-(2'3'-dihydroxypropyl)amino-2-nitro-benzol | 0,56 |
| 1,4-Bis-[(2',3'-dihydroxypropyl)amino]-5-chlor-2-nitro-benzol | 0,24 |
| 4-[(4'-Aminophenyl)-(4'-imino-2',5'-cyclohexadien-1'-yliden)-methyl]-2-methyl-aminobenzol-Hydrochlorid | 0,05 |
| [8-[(p-Aminophenyl)azo]-7-hydroxy-2-naphthyl]-trimethylammoniumchlorid | 0,30 |
| Wasser | ad 100,00 |

Anhand von Messungen mit dem Reflektionsfarbmeßgerät Chromameter II von Minolta wurde festgestellt, daß die Tönung im Waschversuch haltbarer war als eine Tönung ohne Vorbehandlung mit Tanninlösung.

### Beispiel 2: Tönung mit Aluminiumsalzzusatz

Blondes Humanhaar, das bereits durch vorausgegangene Haarbehandlungen oxidativ leicht geschädigt war, wurde mit Tönungsmittel 2 rot gefärbt.

| **Tönungsmittel 2 (rote Tönung)** | **Gew.-%** |
|---|---|
| Fettalkohol | 1,50 |
| Cetyltrimethylammoniumchlorid | 1,00 |
| Ethanol | 8,00 |
| 1-Amino-5-chlor-4-(2',3'-dihydroxypropyl)amino-2-nitro-benzol | 0,35 |
| 1,4-Bis-[(2',3'-dihydroxypropyl)amino]-5-chlor-2-nitro-benzol | 0,15 |
| 4-[(4-Aminophenyl)-(4'-imino-2',5'-cyclohexadien-1'-yliden)-methyl]-2-methyl-aminobenzol-Hydrochlolid | 0,05 |
| 1-(2'-Methoxyphenylazo)-2-hydroxy-7-trimethyl-ammoniumnaphthalene Chloride | 0,40 |
| 4-Hydroxypropylamino-3-nitrophenol | 0,35 |
| Hydroxyethyl-2-nitro-p-toluidin | 0,60 |
| KAl(SO₄)₂ + 12 H₂O | 2,00 |
| Wasser | ad 100,00 |

Anhand von Messungen mit dein Reflektionsfarbmeßgerät Chromameter II von Minolta wurde festgestellt, daß die Tönung im Waschversuch haltbarer war als eine Tönung ohne Alaunzusatz.

### Beispiel 3: Tönung mit Nachbehandlung

Blondes Humanhaar, das bereits durch vorausgegangene Haarbehandlungen oxidativ leicht geschädigt war, wurde mit Tönungsmittel 3 dunkelbraun gefärbt.

| **Tönungsmittel 3** (dunkelbraune Tönung) | **Gew.-%** |
|---|---|
| Fettalkohol | 1,10 |
| Natriumlaurylsulfat | 0,35 |
| Ethanol | 5,00 |
| 1-(2'-Hydroxyethyl)amino-2-nitro-4-bis-(2''-hydroxyethyl)aminobenzol | 0,60 |
| Hydroxyethyl-2-nitro-p-toluidin | 0,09 |
| 4,N-Ethyl,N-(2'-hydroxethyl)amino-1-(2''-hydroxyethyl)amino-2-nitrobenzol | 0,20 |
| 1-Methylamino-4-(2'-hydroxyethyl)amino-anthrachinon | 0,10 |
| 1-(2'-Ureidoethyl)amino-4-nitrobenzol | 0,05 |
| 4-Amino-([4'-bis-(2'-hydroxyethyl)amino]-azobenzol | 0,15 |
| 3-Hydroxy-4-[(2'-hydroxy-1'-naphthyl)azo]-7-nitro-1-naphthalin-sulfonsäure-Natriumsalz | 0,10 |
| Wasser | ad 100,00 |

Anschließend wurde das getönte Haar mit einem Conditioniermittel (pH = 2) bestehend aus
- 4,0%: Cetearylalkohol,
- 0,5%: Trimethylhexadecylammoniumchlorid,
- 1,0%: Aluminiumformiat,
- ad 100,0%: Wasser

2 Minuten behandelt. Anhand von Messungen mit dem Reflektionsfarbmeßgerät Chromameter II von Minolta wurde festgestellt, daß die Tönung im Waschversuch haltbarer war als eine Tönung, die mit einem Aluminiumsalz-freien Conditionierer nachbehandelt wurde.

## Patentansprüche

1. Verwendung von natürlichen oder synthetischen, organischen oder anorganischen Gerbstoffen in Haarbehandlungsmitteln zur Verlängerung der Haltbarkeit von Haartönungen.

2. Verwendung nach Anspruch 1, **dadurch gekennzeichnet**, daß das Haarbehandlungsmittel als natürlichen Gerbstoff einen sich vom Catechin ableitenden, kondensierten Gerbstoff oder einen sich von der Gallussäure ableitenden, hydrolysierbaren Gerbstoff enthält.

3. Verwendung nach Anspruch 1, **dadurch gekennzeichnet**, daß das Haarbehandlungsmittel als synthetischen Gerbstoff ein als Syntan bezeichnetes, durch Sulfonierung löslich gemachtes Aldehyd-Kondensationsprodukt eines Aromaten vom Typ des Phenols, Kresols, Naphthols oder Naphthalins oder ein Harnstoff oder Melamin-Kondensationsprodukt enthält.

4. Verwendung nach Anspruch 1, **dadurch gekennzeichnet**, daß es als anorganischen Gerbstoff ein lösliches Chrom(III)-, Aluminium-, Zirkonium- oder Eisensalz oder ein Polyphosphat enthält.

5. Verwendung nach Anspruch 1, **dadurch gekennzeichnet**, daß das Haarbehandlungsmittel den Gerbstoff in einer Menge von 0,01 bis 5,00 Gewichtsprozent enthält.

6. Verwendung nach den Ansprüchen 1 bis 5, **dadurch gekennzeichnet**, daß das Haarbehandlungsmittel als eine wäßrige, wäßrig-alkoholische oder alkoholische Lösung, als Emulsion, als Shampoo, als Gel oder als Aerosol eingesetzt wird.
